# EUROPEAN PATENT APPLICATION

(11) **EP 4 691 478 A1**
(43) Date of publication of application: **11.02.2026**
(21) Application number: 25762445.2
(22) Date of filing: 14.02.2025
(51) Int. Cl.: A61K 35/32, A61K 41/00, A61K 47/69, A61K 38/39, A61K 9/00, A61P 19/02, C12N 5/077

(54) **PHARMACEUTICAL COMPOSITION FOR TREATING CARTILAGE DISEASES, COMPRISING, AS ACTIVE INGREDIENT, MAGNETIC NANOPARTICLE-EMBEDDED NASAL SEPTAL CHONDROCYTES**

(30) Priority: 29.02.2024 KR 20240029917
(71) Applicant: The Catholic University Of Korea Industry-Academic Cooperation Foundation, Seocho-gu Seoul 06591 (KR)
(72) Inventor: KIM, Man Soo, Seoul 05571 (KR); KIM, Sung Won, Seoul 06001 (KR); CHUN, Jeongho, Seoul 05661 (KR); HAN, Ahreum, Seoul 06981 (KR)
(74) Representative: Dehns
(86) International application number: PCT/KR2025/099383
(87) International publication number: WO 2025/183530

(57) **Abstract**

The present invention relates to a pharmaceutical composition for treating cartilage damage disease, the pharmaceutical composition comprising magnetic nanoparticle-internalized nasal septum chondrocytes as an active ingredient. In addition, the present invention established a method of incorporating magnetic nanoparticles into nasal septum chondrocytes and a method of producing spheroids by culturing the magnetic nanoparticle-internalized nasal septum chondrocytes. The magnetic nanoparticle-internalized nasal septum chondrocytes manufactured by the methods of the present invention, and spheroids produced therefrom were confirmed to exhibit cartilage damage treatment activity while maintaining mobility in a magnetic field. In addition, since the activity is maintained even when the magnetic nanoparticle-internalizednasal septum chondrocytes and nasal septum chondrocytes are mixed to produce spheroids, the addition ratio of the magnetic nanoparticles can be significantly reduced, so that it can be usefully utilized as a composition for treating cartilage damage disease with excellent therapeutic effects without causing side effects.

## Description

### [Technical Field]

The present invention relates to a pharmaceutical composition for treating a cartilage disease, comprising nasal septum chondrocytes internalized with magnetic nanoparticles as an active ingredient.

This application claims priority to and the benefit of Korean Patent Application No. 10-2024-0029917, filed on Febuary 29, 2024, the disclosure of which is incorporated herein by reference in its entirety.

### [Background Art]

Articular cartilage is composed of dense, elastic connective tissue and is located at several points of attachment to the skeleton. Cartilage is connected to bone and its surface is in contact with other cartilage as a connecting part. Cartilage is an avascular tissue that is not innervated and is composed of basic cartilage cells, usually of a single-cell type, and is synthesized into an extracellular matrix (ECM).

Articular cartilage treatment remains an unsolved problem in modern medicine, and although treatment methods exist, many problems remain. One of the primary issues is that articular cartilage has a limited self-repair capacity. To solve these problems, regenerative medicine began to be developed, and cartilage tissue engineering repairs tissue through biological replacement.

The autologous chondrocyte implantation (ACI) technique is used when a large area of the articular cartilage surface is damaged. The autologous cartilage cells to be transplanted are obtained by enzymatically separating a small biopsy of healthy articular cartilage. However, the naturally occurring autologous cartilage cells that are generally known have limitations.

Chondrocyte redifferentiation is limited when expanded in vitro, and the chondrogenic ability decreases with the age of the donor. In addition, cell proliferation is important due to the characteristics of autologous chondrocyte implantation that uses a large number of cells. However, articular cartilage cells are known to have a very low proliferation ability. Human nasal septum chondrocytes can complement the problems of human articular cartilage cells. Human nasal septum chondrocytes have a higher proliferation rate than human articular cartilage cells, and their chondrogenic capacity is also high both in vitro and in vivo and does not decrease with the age of a donor.

However, while the development of a treatment for joint damage using nasal septum chondrocytes with such excellent effects is insufficient, the clinical demand for complete regeneration of damaged cartilage is rapidly increasing, and this demand is expected to increase further as we enter an aging society.

### [Disclosure]

### [Technical Problem]

The present invention is directed to providing a pharmaceutical composition for preventing or treating a cartilage damage disease, comprising magnetic nanoparticle-internalized nasal septum chondrocytes (NSCs) as an active ingredient.

Another objective of the present invention is to provide a kit for preventing or treating cartilage damage disease, comprising nasal septum chondrocytes (NSCs) internalized with magnetic nanoparticles.

Yet another objective of the present invention is to provide a method for producing nasal septum chondrocytes (NSCs) internalized with magnetic nanoparticles, the method comprising:
(S1) producing nasal septum chondrocytes (NSCs) by treating nasal septum cartilage tissue, isolated from nasal septum tissue, with type 2 collagenase; and
(S2) culturing the nasal septum chondrocytes and additionally adding magnetic nanoparticles thereto.

Yet another objective of the present invention is to provide a method for producing magnetic nanoparticle-internalized nasal septum chondrocyte spheroids for treating cartilage damage disease, the method comprising:
(S1) producing magnetic nanoparticle-internalized nasal septum chondrocytes by culturing nasal septum chondrocytes (NSCs), isolated from nasal septum tissue, and magnetic nanoparticles; and
(S2) adding the nasal septum chondrocytes and the magnetic nanoparticle-internalized nasal septum chondrocytes to a medium for culturing them in a spheroid shape.

However, technical problems to be solved in the present invention are not limited to the above-described problems, and other problems which are not described herein will be fully understood by those of ordinary skill in the art from the following descriptions.

### [Technical Solution]

The present invention provides a pharmaceutical composition for preventing or treating cartilage damage disease, comprising magnetic nanoparticle-internalized nasal septum chondrocytes (NSCs) as an active ingredient.

In one embodiment of the present invention, the magnetic nanoparticle-internalizednasal septum chondrocytes may be spheroids, but are not limited thereto.

In one embodiment of the present invention, the magnetic nanoparticle-internalized nasal septum chondrocytes may express collagen type 2, but are not limited thereto.

In one embodiment of the present invention, in the magnetic nanoparticle-internalized nasal septum chondrocytes, the magnetic nanoparticles may be added in an amount of more than 0 µg to 800 µg per 1 × 10^6 nasal septum chondrocytes, but are not limited thereto.

In one embodiment of the present invention, the spheroids may be a mixture of nasal septum chondrocytes and the magnetic nanoparticle-internalized nasal septum chondrocytes, but are not limited thereto.

In one embodiment of the present invention, the number ratio of the nasal septum chondrocytes : the magnetic nanoparticle-internalized nasal septum chondrocytes may be (5 to 7) : (3 to 5), but is not limited thereto.

In one embodiment of the present invention, the magnetic nanoparticle-internalized nasal septum chondrocytes may maintain mobility in a magnetic field regardless of the concentration of the magnetic nanoparticles, but are not limited thereto.

In one embodiment of the present invention, the composition may be characterized by one or more selected from the group consisting of the following, but is not limited thereto:
a) inducing a restoration effect of a hypoechoic substance at the site of cartilage damage;
b) exerting a cartilage regeneration effect; and
c) exhibiting an anti-inflammatory effect.

In one embodiment of the present invention, the composition may be for injection, but is not limited thereto.

In one embodiment of the present invention, the composition may be administered by a method selected from the group consisting of arthroscopic injection and intra-articular injection, but is not limited thereto.

In one embodiment of the present invention, the cartilage damage disease may be one or more selected from the group consisting of osteoarthritis, degenerative arthritis, articular cartilage defect, rheumatoid arthritis, fracture, meniscus injury, muscle injury, ligament injury, plantar fasciitis, lateral epicondylitis, calcific myositis, traumatic joint or cartilage injury, and nonunion of fractures, but is not limited thereto.

The present invention provides a kit for preventing or treating cartilage damage disease, comprising nasal septum chondrocytes (NSCs) internalized with magnetic nanoparticles.

The present invention provides a method of producing nasal septum chondrocytes internalized with magnetic nanoparticles, the method comprising:
(S1) producing nasal septum chondrocytes (NSCs) by treating nasal septum cartilage tissue, isolated from nasal septum tissue, with type 2 collagenase; and
(S2) culturing the nasal septum chondrocytes and additionally adding magnetic nanoparticles thereto.

The present invention provides a method of producing magnetic nanoparticle-internalized nasal septum chondrocyte spheroids for treating cartilage damage disease, the method comprising:
(S1) producing magnetic nanoparticle-internalized nasal septum chondrocytes by culturing nasal septum chondrocytes (NSCs), isolated from nasal septum tissue, and magnetic nanoparticles; and
(S2) adding the nasal septum chondrocytes and the magnetic nanoparticle-internalized nasal septum chondrocytes to a medium for culturing them in a spheroid shape.

The present invention also provides a method of preventing or treating cartilage damage disease, the method comprising: administering a composition comprising the magnetic nanoparticle-internalized nasal septum chondrocytes (NSCs) as an active ingredient in a pharmaceutically effective amount to a subject in need thereof.

The present invention also provides a use of a composition comprising magnetic nanoparticle-internalized nasal septum chondrocytes (NSCs) as an active ingredient for preventing or treating cartilage damage disease.

The present invention also provides a use of a composition comprising the magnetic nanoparticle-internalized nasal septum chondrocytes (NSCs) as an active ingredient for producing a preparation for preventing or treating cartilage damage disease.

### [Advantageous Effects]

According to the pharmaceutical composition for treating cartilage damage disease comprising nasal septum chondrocytes internalized with magnetic nanoparticles as an active ingredient, a method of incorporating magnetic nanoparticles into nasal septum chondrocytes and a method of culturing the nasal septum chondrocytes internalized with magnetic nanoparticles to produce spheroids were established. It was confirmed that the nasal septum chondrocytes internalized with magnetic nanoparticles and the spheroids prepared therefrom by the method of the present invention exhibited therapeutic activity for cartilage damage while maintaining mobility in a magnetic field. In addition, even when spheroids are produced by mixing nasal septum chondrocytes internalized with magnetic nanoparticles and nasal septum chondrocytes, the above-described activity is maintained, and thus the amount of magnetic nanoparticles added may be significantly reduced, making the composition useful as a therapeutic composition for cartilage damage disease with excellent therapeutic effects without causing side effects.

### [Brief Description of the Drawings]

FIG. 1A is a schematic diagram illustrating a protocol for isolating and culturing nasal septum chondrocytes (hereinafter, hNCs) from nasal septum cartilage tissue, as established in the present invention.
FIG. 1B shows the morphological characteristics of hNCs isolated and cultured from nasal septum cartilage tissue, indicating that no morphological changes occurred through the hNC isolation and culture protocol of the present invention.
FIG. 1C is a graph comparing the expression levels of collagen type 2 in hNCs and hNC spheroids derived therefrom, indicating that the expression level of collagen type 2 was significantly increased in the hNC spheroids.
FIGS. 2A and 2B show that magnetic nanoparticles were successfully internalized into hNCs to produce hNCs internalized with magnetic nanoparticles (hNC cell bots), as confirmed by microscopic and TEM analyses, respectively.
FIG. 3 shows whether the cell proliferation ability of hNCs internalized with magnetic nanoparticles changes depending on the concentration of magnetic nanoparticles, indicating that there was no change in the cell proliferation ability of the hNCs.
FIGS. 4A and 4B respectively show that hNC spheroids can be formed and retain mobility regardless of the concentration when magnetic nanoparticles are internalized at varying concentrations.
FIG. 5A is a schematic diagram illustrating a method for producing mixed hNC spheroid cell bots to reduce the amount of magnetic nanoparticles.
FIG. 5B shows the mobility in a magnetic field of mixed hNC spheroid cell bots according to the mixing ratio of hNC spheroid cell bots.
FIG. 6A shows an osteoarthritis animal model established to evaluate the therapeutic effect of hNC spheroid cell bots on cartilage damage.
FIG. 6B shows the results of clinical symptom analysis after transplantation of hNC spheroid cell bots into the osteoarthritis animal model, indicating changes in feed intake and body weight.
FIG. 6C shows the results of tomography after transplantation of hNC spheroid cell bots into the osteoarthritis animal model, presenting representative Micro-CT images for each subject.
FIG. 6D shows the results of histopathological analysis after transplantation of hNC spheroid cell bots into the osteoarthritis animal model, presenting histopathological images for each subject after H&E staining.
FIG. 6E shows the results of a scoring analysis based on the histopathological analysis.

### [Best Modes of the Invention]

The present invention provides a pharmaceutical composition for preventing or treating cartilage damage disease, comprising magnetic nanoparticle-internalized nasal septum chondrocytes (NSCs) as an active ingredient.

As used in the present invention, the term "chondrocyte" refers to a cell present in lacunae within the cartilage matrix and responsible for synthesizing and secreting the cartilage matrix, having well-developed rough endoplasmic reticulum and Golgi apparatus. The shape of the chondrocyte corresponds to that of the lacunae; it appears elongated oval or flattened under the perichondrium and in the superficial layer of articular cartilage, and appears hemispherical or polygonal in the deep zone. Polysaccharide-protein complexes are bound to the cell membrane of the chondrocyte. These complexes are spatially associated with polysaccharides or fibers in the matrix, thereby suspending the chondrocyte within the matrix. In the present invention, the term "chondrocyte" also encompasses chondroprogenitor cells, which are cells already committed to chondrogenic differentiation.

As used in the present invention, the term "nasal septum chondrocyte" refers to a chondrocyte that constitutes the anterior portion of the nasal septum, which is the partition wall dividing the left and right nasal cavities. The nasal septum chondrocytes of the present invention may be isolated from nasal septum cartilage tissue discarded during septoplasty, which is one of the most frequently performed surgeries, or from biopsy tissue obtained under local anesthesia, and have the advantage of being derived from a non-weight-bearing donor site with no complications under local anesthesia. Although there are no reports on the number of chondrocytes isolated from nasal septum tissue, a greater number of cells can be secured compared to articular chondrocytes, and chondrocytes isolated from the hyaline cartilage of the nasal septum exhibit higher proliferative capacity and superior ability to produce cartilage-specific extracellular matrix during in vitro culture compared to articular chondrocytes.

The present invention also provides a method for isolating and culturing nasal septum chondrocytes, the method comprising the steps of:
(S1) cutting the isolated nasal septum cartilage and treating it with type 2 collagenase;
(S2) culturing the cartilage treated with type 2 collagenase, filtering, and harvesting the cells; and
(S3) subculturing the harvested chondrocytes up to passage 3.

As used in the present invention, the term "magnetic particles" refers to magnetized materials that may be applied in various fields due to their structural and magnetic properties, such as MRI contrast agents, biomedical diagnostic devices, anti-counterfeiting inks, and control devices within speaker components. In everyday life, which involves numerous devices powered by electricity, magnetic particles allow for magnetic control. Examples comprise iron oxides (Fe₂O₃, Fe₃O₄); ferrites, in which one Fe atom in Fe₃O₄ is substituted with another magnetic element (e.g., CoFe₂O₄, MnFe₂O₄); and magnetic alloys (e.g., FePt, CoPt), which are formed by alloying magnetic atoms with noble metals to improve conductivity and stability and to address oxidation issues. In practical applications, magnetic particles are not used in powder form, but rather dispersed in liquids, forming a ferrofluid. When a magnetic field is applied to such a ferrofluid, the field induces the fluid to rise sharply along magnetic field lines only in the magnetized regions. From an industrial application perspective, the use of magnetic fields offers a cost-effective approach and enables flexible control by adjusting the intensity and direction of the magnetic field.

As used in the present invention, the term "magnetic nanoparticles" refers to ferromagnetic magnetic particles having a nanoscale size in an extremely fine range, and are generally known to have a size ranging from several nanometers to several hundred nanometers. The magnetic nanoparticles of the present invention are used in a broad sense to comprise magnetic particles that may be referred to as magnetic nanoparticles by those skilled in the art. For example, in the present invention, the magnetic nanoparticles may have a size of 1 to 500 nm, 1 to 400 nm, 1 to 300 nm, 1 to 200 nm, 1 to 100 nm, 30 to 500 nm, 30 to 400 nm, 30 to 300 nm, 30 to 200 nm, 30 to 100 nm, 50 to 500 nm, 50 to 400 nm, 50 to 300 nm, 50 to 200 nm, 50 to 100 nm, 70 to 500 nm, 70 to 400 nm, 70 to 300 nm, 70 to 200 nm, 70 to 100 nm, 90 to 500 nm, 90 to 400 nm, 90 to 300 nm, 90 to 200 nm, or 90 to 100 nm; In one embodiment of the present invention, magnetic nanoparticles having a size of 100 nm were used, but the present invention is not limited thereto.

In the present invention, fluidMAG-DX (4104-1 (1 ml); 4104-5 (5 ml)) from Chemicell was used, but the present invention is not limited thereto. The characteristics of the magnetic nanoparticles used in the present invention are as follows:
Product description: Aqueous dispersion of magnetic nanoparticles; (2) Weigh of Volume : 25 mg/ml; (3) Core : Maghemite; (4) Matrix : Dextran; (5) Size : 100nm; (6) Number of particles : ~1.8 x 10^15/g; (7) Density : ~1.25g/cm^3; (8) Type of Magnetization : Superparamagnetic; (9) Functional Group : Hydroxyl groups; Autoclaved; (10) Storage Buffer : ddH₂O; (11) Storage : 4~8°C, (12) Do not freeze; (13) Expiry date : Two years after production date.

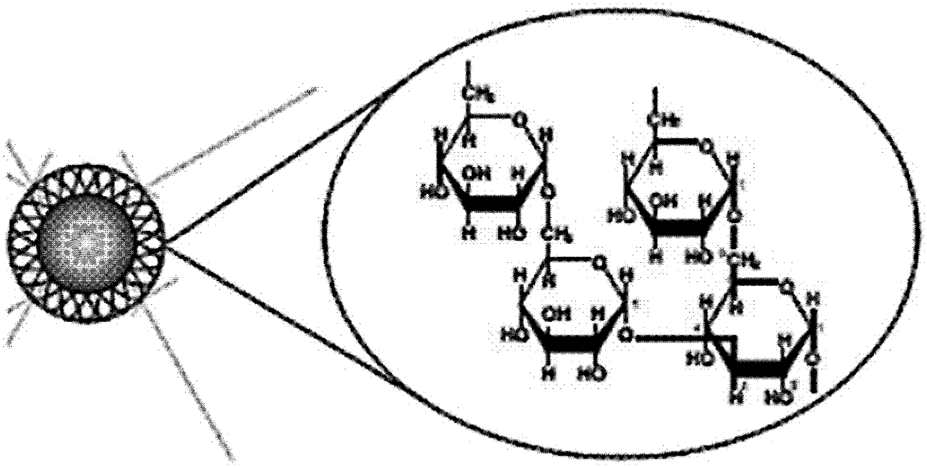

As used in the present invention, the term "damage" refers to any phenomenon in which the normal structure of a tissue is morphologically disrupted regardless of the cause, and the term "cartilage damage" may refer to such damage occurring in cartilage.

In one embodiment of the present invention, the magnetic nanoparticle-internalized nasal septum chondrocytes may be spheroids, but are not limited thereto.

As used in the present invention, the term "spheroid" refers to a three-dimensional cell aggregate capable of self-organization. A spheroid is a miniature and simplified form of a three-dimensional ex vivo organ that mimics the anatomical structure of real tissue, and may be used for various drug screening applications and disease models. The term "spheroid" generally refers to a three-dimensional structure in which cells are aggregated to a degree that the cross-section may appear circular or elliptical; however, such morphology should be interpreted in consideration of the properties of the cells or cell aggregates, and it is apparent that it does not necessarily refer to a perfectly spherical or ideal spheroid form.

In one embodiment of the present invention, the magnetic nanoparticle-internalized nasal septum chondrocytes may express collagen type 2, but are not limited thereto.

As used in the present invention, with respect to "collagen type 2," collagen refers to a scleroprotein primarily present in animal bones and skin, and also distributed in cartilage, organ membranes, hair, and the like. It is also known as collagens and exists in fibrous solid form. Under an electron microscope, collagen has a complex cross-striated structure, and it is insoluble in water, dilute acid, and dilute alkali, but becomes gelatin and dissolves when boiled. There are six known types of collagen, and collagen type 2 is classified as one of them and is known to be a major component of cartilage.

In the present invention, it was confirmed that collagen type 2 was expressed not only in nasal septum chondrocytes isolated and obtained from nasal septum tissue, but also in spheroid forms produced by culturing the nasal septum chondrocytes. The spheroid form of nasal septum chondrocytes may exhibit a higher level of collagen type 2 expression compared to the nasal septum chondrocytes themselves, but is not limited thereto.

In one embodiment of the present invention, in the magnetic nanoparticle-internalizednasal septum chondrocytes, the magnetic nanoparticles may be added in an amount of more than 0 µg to 800 µg per 1 × 10^6 nasal septum chondrocytes, but are not limited thereto.

In the present invention, the amount of magnetic nanoparticles to be added may be more than 0 µg and not more than 800 µg, more than 0 µg and not more than 700 µg, more than 0 µg and not more than 600 µg, more than 0 µg and not more than 500 µg, more than 0 µg and not more than 400 µg, 30 µg to 800 µg, 30 µg to 700 µg, 30 µg to 600 µg, 30 µg to 500 µg, 30 µg to 400 µg, 60 µg to 800 µg, 60 µg to 700 µg, 60 µg to 600 µg, 60 µg to 500 µg, 60 µg to 400 µg, 80 µg to 800 µg, 80 µg to 700 µg, 80 µg to 600 µg, 80 µg to 500 µg, 80 µg to 400 µg, 100 µg to 800 µg, 100 µg to 700 µg, 100 µg to 600 µg, 100 µg to 500 µg, or 100 µg to 400 µg, but is not limited thereto.

In one embodiment of the present invention, the spheroids may be a mixture of nasal septum chondrocytes and the magnetic nanoparticle-internalized nasal septum chondrocytes, but are not limited thereto.

In one embodiment of the present invention, the number ratio of the nasal septum chondrocytes : the magnetic nanoparticle-internalized nasal septum chondrocytes may be (5 to 7) : (3 to 5), but is not limited thereto.

In one embodiment of the present invention, the magnetic nanoparticle-internalized nasal septum chondrocytes may maintain mobility in a magnetic field regardless of the concentration of the magnetic nanoparticles, but are not limited thereto. That is, in the present invention, the nasal septum chondrocytes internalized with magnetic nanoparticles may maintain mobility in a magnetic field regardless of whether they are in the form of spheroids and regardless of the concentration of the magnetic nanoparticles.

In one embodiment of the present invention, the composition may be characterized by one or more selected from the group consisting of the following, but is not limited thereto:
a) inducing a restoration effect of a hypoechoic substance at the site of cartilage damage;
b) exerting a cartilage regeneration effect; and
c) exhibiting an anti-inflammatory effect.

In one embodiment of the present invention, the composition may be for injection, but is not limited thereto.

In one embodiment of the present invention, the composition may be administered by a method selected from the group consisting of arthroscopic injection and intra-articular injection, but is not limited thereto.

The composition of the present invention is basically intended for use as an injectable formulation, but the method of injection may comprise all methods generally used in the art. Therefore, the composition of the present invention may be administered by methods such as injection using arthroscopy or intra-articular injection. In addition, the composition may be administered not only through injection therapy without arthroscopy, but also through direct administration methods such as injection after surgical incision, but is not limited thereto.

In one embodiment of the present invention, the cartilage damage disease may be one or more selected from the group consisting of osteoarthritis, degenerative arthritis, articular cartilage defect, rheumatoid arthritis, fracture, meniscus injury, muscle injury, ligament injury, plantar fasciitis, lateral epicondylitis, calcific myositis, traumatic joint or cartilage injury, and nonunion of fractures, but is not limited thereto.

The pharmaceutical composition according to the present invention may be used by being formulated, according to commonly used methods, into a form such as powders, granules, sustained-release-type granules, enteric granules, liquids, eye drops, elixirs, emulsions, suspensions, spirits, troches, aromatic water, lemonades, tablets, sustained-release-type tablets, enteric tablets, sublingual tablets, hard capsules, soft capsules, sustained-release-type capsules, enteric capsules, pills, tinctures, soft extracts, dry extracts, fluid extracts, injections, capsules, perfusates, or a preparation for external use, such as plasters, lotions, pastes, sprays, inhalants, patches, sterile injectable solutions, or aerosols. The preparation for external use may have a formulation such as creams, gels, patches, sprays, ointments, plasters, lotions, liniments, pastes, or cataplasmas.

As the carrier, the excipient, and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used.

For formulation, commonly used diluents or excipients such as fillers, thickeners, binders, wetting agents, disintegrants, and surfactants are used.

As additives of tablets, powders, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and *Primojel*^{®}; and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol, and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ion-exchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution, and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid may be used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, and sodium carboxymethylcellulose may be used.

In syrups according to the present invention, a white sugar solution, other sugars or sweeteners, and the like may be used, and as necessary, a fragrance, a colorant, a preservative, a stabilizer, a suspending agent, an emulsifier, a viscous agent, or the like may be used.

In emulsions according to the present invention, purified water may be used, and as necessary, an emulsifier, a preservative, a stabilizer, a fragrance, or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, HPMC 2910, and the like may be used, and as necessary, a surfactant, a preservative, a stabilizer, a colorant, and a fragrance may be used.

Injections according to the present invention may comprise: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO₃) carbon dioxide gas, sodium metabisulfite (Na₂S₂O₅), sodium sulfite (Na₂SO₃), nitrogen gas (N₂), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; a pain relief agent such as benzyl alcohol, chlorobutanol, procaine hydrochloride, glucose, and calcium gluconate; and suspending agents such as sodium CMC, sodium alginate, Tween 80, and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, lanette wax, glycerol monostearate, Tween or span, imhausen, monolan(propylene glycol monostearate), glycerin, Adeps solidus, buytyrum Tego-G, cebes Pharma 16, hexalide base 95, cotomar, Hydrokote SP, S-70-XXA, S-70-XX75(S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro OSI, OSIX, A, B, C, D, H, L, suppository base IV types AB, B, A, BC, BBG, E, BGF, C, D, 299, suppostal N, Es, Wecoby W, R, S, M, Fs, and tegester triglyceride matter (TG-95, MA, 57) may be used.

Solid preparations for oral administration comprise tablets, pills, powders, granules, capsules, and the like, and such solid preparations are formulated by mixing the composition with one or more excipient, e.g., starch, calcium carbonate, sucrose, lactose, gelatin, and the like. In addition to simple excipients, lubricants such as magnesium stearate and talc are also used.

Examples of liquid preparations for oral administration comprise suspensions, liquids for internal use, emulsions, syrups, and the like, and these liquid preparations may comprise, in addition to simple commonly used diluents, such as water and liquid paraffin, various types of excipients, for example, a wetting agent, a sweetener, a fragrance, a preservative, and the like. Preparations for parenteral administration comprise an aqueous sterile solution, a non-aqueous solvent, a suspension, an emulsion, a freeze-dried preparation, and a suppository. Non-limiting examples of the non-aqueous solvent and the suspension comprise propylene glycol, polyethylene glycol, a vegetable oil such as olive oil, and an injectable ester such as ethyl oleate.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors comprising types of diseases of patients, the severity of disease, the activity of drugs, sensitivity to drugs, administration time, administration route, excretion rate, treatment period, and simultaneously used drugs, and factors well known in other medical fields.

The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

The pharmaceutical composition of the present invention may be administered to a subject via various routes. All administration methods can be predicted, and the pharmaceutical composition may be administered via, for example, subcutaneous injection, intraperitoneal injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, or the like.

The pharmaceutical composition of the present invention is determined depending on the type of a drug, which is an active ingredient, along with various related factors such as a disease to be treated, administration route, the age, gender, and body weight of a patient, and the severity of diseases.

As used herein, the "subject" refers to a subject in need of treatment of a disease, and more specifically, refers to a mammal such as a human or a non-human primate, a mouse, a rat, a dog, a cat, a horse, and a cow.

As used herein, the "administration" refers to providing a subject with a predetermined composition of the present invention by using an arbitrary appropriate method.

The term "prevention" as used herein means all actions that inhibit or delay the onset of a target disease. The term "treatment" as used herein means all actions that alleviate or beneficially change a target disease and abnormal metabolic symptoms caused thereby via administration of the pharmaceutical composition according to the present invention. The term "alleviation" as used herein means all actions that reduce the degree of parameters related to a target disease, e.g., symptoms via administration of the composition according to the present invention.

The present invention also provides a method of preventing or treating cartilage damage disease, the method comprising: administering a composition comprising the magnetic nanoparticle-internalized nasal septum chondrocytes (NSCs) as an active ingredient in a pharmaceutically effective amount to a subject in need thereof.

The present invention also provides a use of a composition comprising magnetic nanoparticle-internalized nasal septum chondrocytes (NSCs) as an active ingredient for preventing or treating cartilage damage disease.

The present invention also provides a use of a composition comprising the magnetic nanoparticle-internalized nasal septum chondrocytes (NSCs) as an active ingredient for producing a preparation for preventing or treating cartilage damage disease.

The present invention provides a kit for preventing or treating cartilage damage disease, comprising nasal septum chondrocytes (NSCs) internalized with magnetic nanoparticles.

In the present invention, the term "kit" refers to a tool that enables the prevention or treatment of cartilage damage disease using the nasal septum chondrocytes internalized with magnetic nanoparticles of the present invention. In this case, the nasal septum chondrocytes internalized with magnetic nanoparticles may be nasal septum chondrocytes, and in one embodiment of the present invention, a culture composition for culturing the cells in the form of spheroids may additionally be included, but is not limited thereto.

In the present invention, the kit may comprise a container, instructions, and the like. The container may serve to package the above substance, and may also serve to store and secure it. The material of the container may take the form of, for example, a bottle, tub, sachet, envelope, tube, or ampoule, and may be formed partially or entirely from plastic, glass, paper, foil, wax, or the like. The container may be equipped with a cap that is initially a part of the container or is completely or partially detachable and attachable to the container by mechanical, adhesive, or other means, and may also be equipped with a stopper that allows access to the contents with a syringe needle. The kit may comprise an external package, and the external package may comprise instructions for use of the components.

The present invention provides a method of producing nasal septum chondrocytes internalized with magnetic nanoparticles, the method comprising:
(S1) producing nasal septum chondrocytes (NSCs) by treating nasal septum cartilage tissue, isolated from nasal septum tissue, with type 2 collagenase; and
(S2) culturing the nasal septum chondrocytes and additionally adding magnetic nanoparticles thereto.

The present invention provides a method of producing magnetic nanoparticle-internalized nasal septum chondrocyte spheroids for treating cartilage damage disease, the method comprising:
(S1) producing magnetic nanoparticle-internalized nasal septum chondrocytes by culturing nasal septum chondrocytes (NSCs), isolated from nasal septum tissue, and magnetic nanoparticles; and
(S2) adding the nasal septum chondrocytes and the magnetic nanoparticle-internalized nasal septum chondrocytes to a medium for culturing them in a spheroid shape.

In step (S1) of the method for producing mixed nasal septum chondrocyte spheroids internalized with magnetic nanoparticles for treating cartilage damage disease according to the present invention, the step of producing nasal septum chondrocytes internalized with magnetic nanoparticles may comprise the above-described method for producing nasal septum chondrocytes internalized with magnetic nanoparticles provided in one embodiment of the present invention, but is not limited thereto.

### [Modes of the Invention]

Hereinafter, preferable examples are presented to aid in the understanding of the present invention. However, these examples are provided merely for easier understanding of the present invention, and the content of the present invention is not limited by these examples.

### [Examples]

### Example 1. Isolation of cartilage cells derived from nasal septum and production of spheroids

### Example 1-1. Isolation and culture of cartilage cells derived from nasal septum

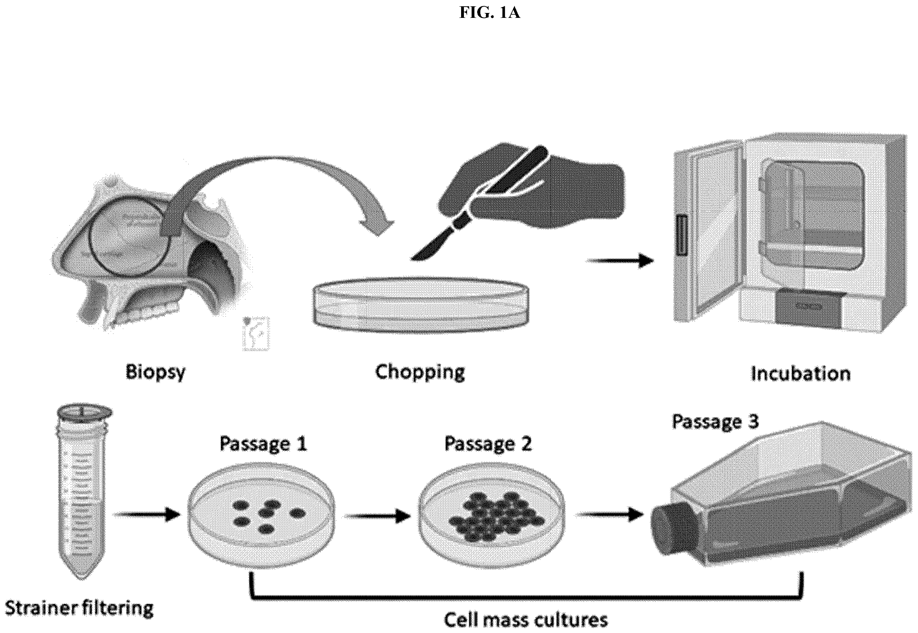

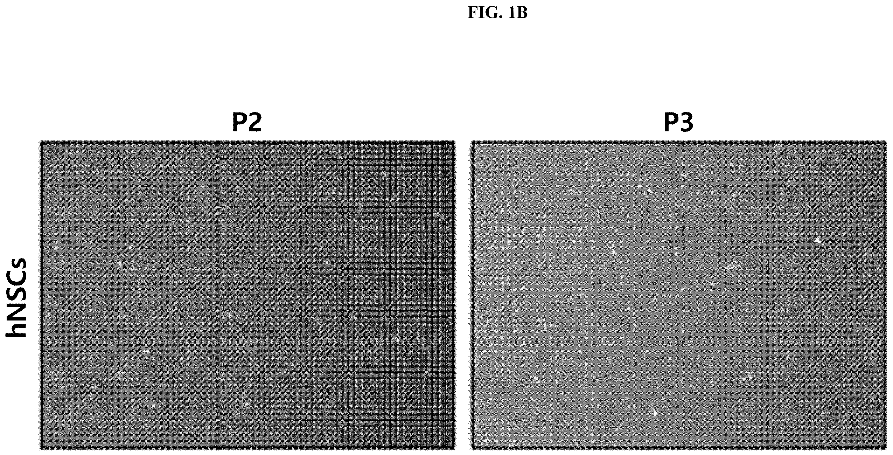

A protocol for isolating cartilage cells derived from human nasal septum (hereinafter referred to as "hNCs") was established by treating human nasal septum cartilage tissue discarded during septoplasty and human nasal septum cartilage tissue obtained during biopsy with type 2 collagenase (FIG. 1A). First, the biopsied human nasal septum cartilage was washed three times with phosphate-buffered saline (PBS) and minced into 1 mm³ in a non-coated dish, and the minced tissue was treated with 0.2% type 2 collagenase in growth media (low DMEM, 10% FBS, 1% PS) and reacted overnight in a cell culture incubator. The isolated hNCs were filtered using a 70 um filter, and then harvested. When the isolated hNCs were subcultured up to passage 3, the characteristic shape of cartilage cells was observed, and there was no significant morphological change in the cells during the cell culture process up to passage 3 (FIG. 1B). Accordingly, a culture process protocol was established.

### Example 1-2. Comparison of Collagen type 2 expression levels in hNCs and hNC spheroids

The protein expression amounts of Collagen type 2 in the hNCs isolated and cultured in Example 1-1 and hNC spheroids produced from the hNCs were compared and analyzed by western blot. Specifically, hNCs were harvested using RIPA buffer, reacted on ice for about 20 minutes, and pelleted down in a 4°C centrifuge for 20 minutes, and only the supernatant was used. Proteins were quantified using the BCA assay and denatured with SDS buffer at 100°C for 5 minutes. The quantified protein samples were electrophoresed at 80 V on a 6% polyacrylamide gel and then transferred to a PVDF membrane. Next, the PVDF membrane was blocked using 5% skim milk and the antibody of interest was attached to confirm.

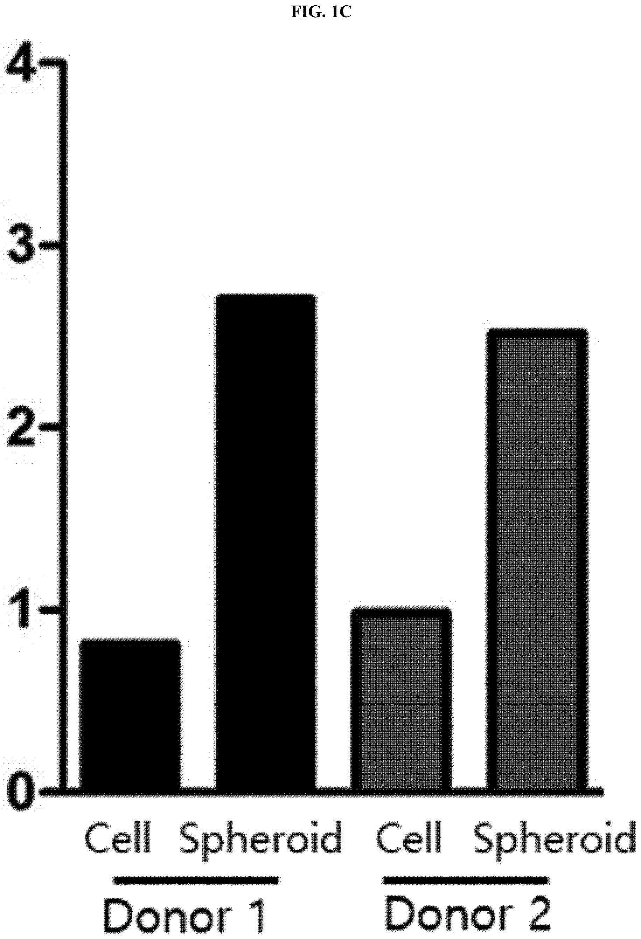

As a result, it was confirmed that the expression level of Collagen type 2, a cartilage-specific marker, was increased in hNC spheroids, compared to hNCs that were in a cell state (FIG. 1C).

### Example 2. Production and verification of hNCs internalized with magnetic nanoparticles

### Example 2-1. Production of hNCs internalized with magnetic nanoparticles

hNCs internalized with magnetic nanoparticles (fluidMAG-DX, 4104-1 (1ml); 4104-5 (5ml), aqueous dispersion of magnetic nanoparticles, Chemicell) were produced. Specifically, 1x10^6 hNCs separated by the method of Example 1-1 were seeded with 10 ml of culture medium in a 100 mm culture dish and then cultured overnight. After confirming that the hNCs were well attached to the culture dish, they were washed twice with DPBS, and magnetic nanoparticles were diluted in the culture medium to the concentration of the magnetic nanoparticles to be internalized (0 µg/ml, 100 µg/ml, 200 µg/ml, and 400 µg/ml), followed by culturing for approximately 3 days.

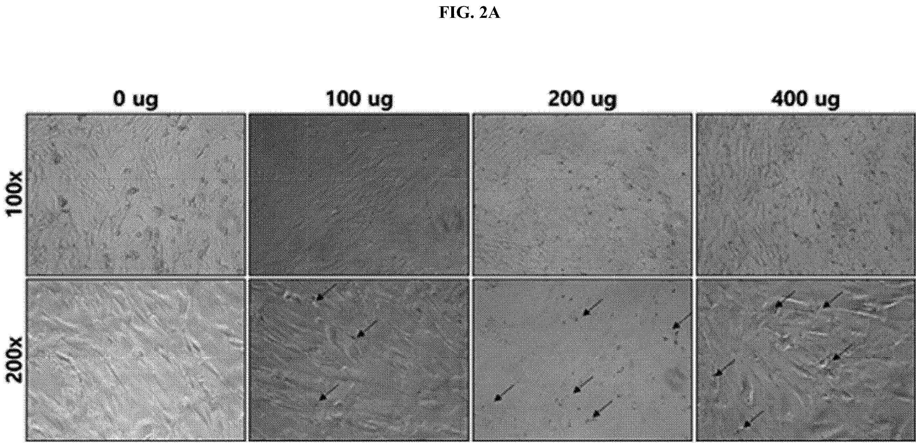

After 3 days, observation under a microscope confirmed that magnetic nanoparticles were successfully internalized into hNCs, as shown in FIG. 2A.

### Example 2-2. Precise analysis of hNCs internalized with magnetic nanoparticles

To precisely analyze the hNCs internalized with the magnetic nanoparticles produced in Example 2-1, a transmission electron microscope (TEM) analysis was performed. Here, hNCs internalized with magnetic nanoparticles were produced using 100 ug/ml of magnetic nanoparticles in the same method as in Example 2-1, and used for analysis. Culture was performed using a confocal dish, and transmission electron microscope analysis was performed. Specifically, a 2.5% GA fixative was preheated to 37°C in a water bath or incubator without using a rinse before cell fixation. A warm 2.5% GA fixative was added in an equal volume to the cell culture medium (vol of fixative/vol of medium = 1/1) along the wall of a 12-well culture plate. Next, a culture plate was rotated 4 to 5 times on a culture table, and the culture plate was left on the culture table for 5 minutes. After labeling with a clear sample identification number, the culture plate was sealed well with parafilm, and kept at 4°C until the existing TEM sample preparation training/service schedule was scheduled.

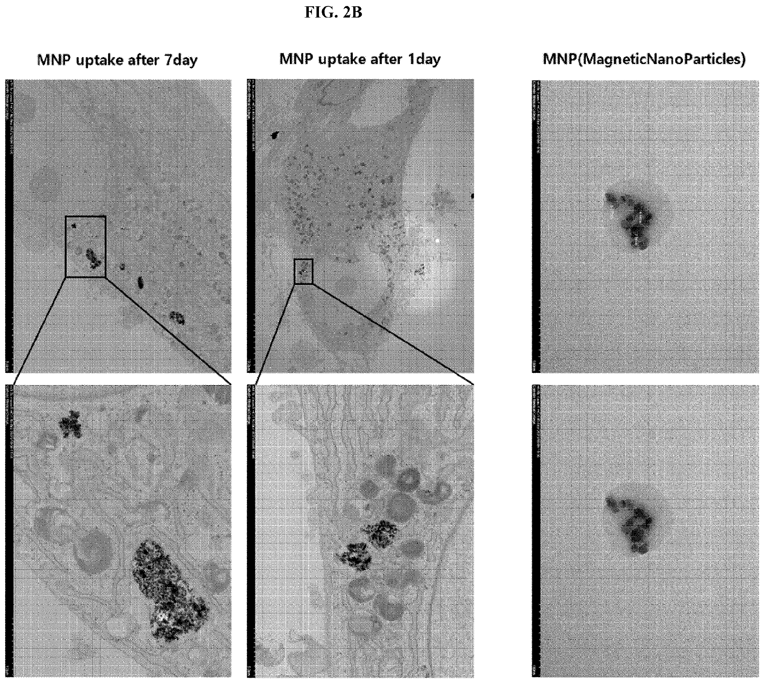

As a result, it was confirmed also by TEM analysis that hNCs internalized with magnetic nanoparticles were successfully produced, similar to Example 2-1 (FIG. 2B).

### Example 3. Determination of effect of magnetic nanoparticles concentration on hNC proliferation ability

The occurrence of abnormalities in the cell proliferation ability of hNCs dependent upon the incorporation concentration of magnetic nanoparticles according to the present invention was analyzed. To this, hNCs were prepared by incorporating magnetic nanoparticles at each concentration of 0 µg/ml, 100 µg/ml, 200 µg/ml, and 400 µg/ml magnetic nanoparticles, respectively, and the cell proliferation ability of the hNCs at each concentration was confirmed on days 1, 3, and 5. Here, cell proliferation ability was analyzed through WST-8. Specifically, cells were prepared in 6-well culture dishes according to culture conditions, and WST-8 solution was diluted with culture medium in a ratio of 10:1 and reacted with the cells at 37°C for 1 h for each time condition, followed by checking their wavelength value at 450 nm.

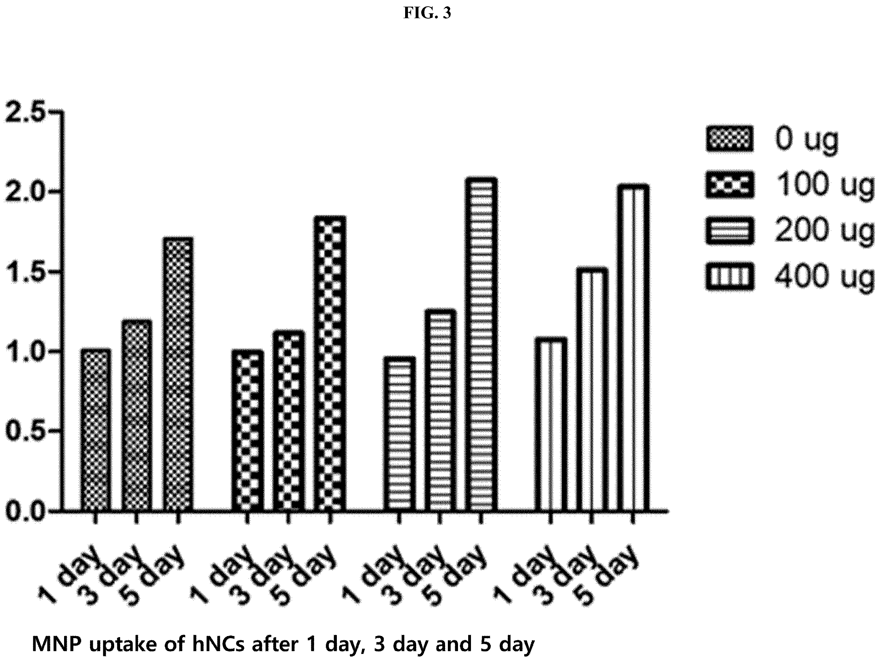

As a result, it was confirmed that the magnetic nanoparticles did not show any effect on the proliferation ability of hNCs regardless of their concentration (FIG. 3).

### Example 4. Production of hNC spheroids internalized with magnetic nanoparticles and confirmation of no relationship between mobility and the amount of magnetic nanoparticles

### Example 4-1. Production of hNC spheroids using different concentrations of magnetic nanoparticles

In Example 3, it was confirmed that the hNCs proliferation ability was maintained regardless of the incorporation concentration of magnetic nanoparticles. In this example, it was analyzed whether the amount of magnetic nanoparticles affects the production of hNC spheroids. Here, the magnetic nanoparticles were used at a concentration of each of 0 µg/ml, 100 µg/ml, 200 µg/ml, and 400 µg/ml, as in Example 3. Specifically, StemFIT 3D-Plastic (H853400-P) was used as a cell culture vessel for culturing cells in the form of spheroids, and all media additions and replacements were performed at the inner corner of StemFIT 3D-Plastic (H853400-P). First, StemFIT 3D-Plastic (H853400-P) was placed on a dish to be cultured, filled with DPBS, and then bubbles were removed by pipetting. After all bubbles were removed, DPBS was suctioned from the corner of the StemFIT 3D-Plastic (H853400-P) using a pipette. Here, care was taken to ensure that all DPBS in the microwells was not removed and bubbles did not form again. To enable cell culture in the wells without forming bubbles in the wells, each well was filled with DPBS, and then filled with the cell culture medium. After removing the DPBS inside the microwells by pipetting once more, suction was performed. The prepared single cell was seeded on StemFIT 3D-Plastic (H853400-P), and the cells were allowed to settle for 10 minutes inside the microwells of StemFIT 3D-Plastic (H853400-P). At this time, if the microwells are placed on the microscope and shaken slightly, floating cells will be observed as the medium shakes. If the cells are washed before they have completely settled, the cells will be lost. Accordingly, in the case of cells other than embryonic stem cells, the size of the spheroids will be reduced, so it was left until all floating cells settled. After 10 minutes, if there were many cells between the wells, the medium was slowly suctioned with a pipette from the inner corner of the StemFIT 3D and the cell culture medium was filled to create surface tension inside the StemFIT 3D-Plastic (H853400-P).

The viability of cells cultured in concave molds was assessed using a live/dead assay kit (Invitrogen, Carlsbad, CA, USA). Specifically, 5 ul of calcein AM solution (Invitrogen) and 20 ul of ethidium Homodimer-1 solution (Invitrogen) were dissolved in 10 ml of DPBS (Gibco-BRL), and then added to the cells. Next, the cells were incubated at 37°C for 20 minutes.

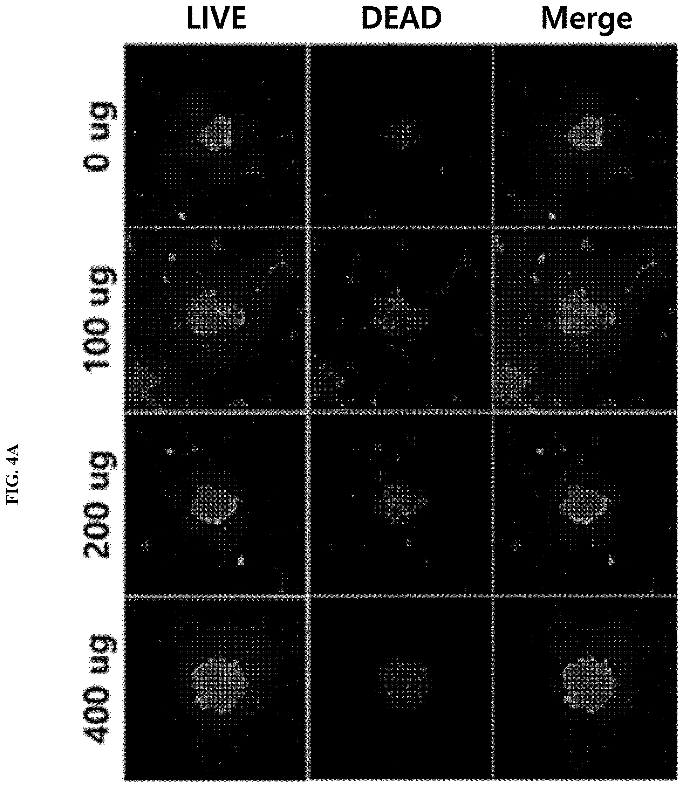

As a result, it was confirmed that although the magnetic nanoparticles were internalized into hNCs under different concentration conditions to produce hNC spheroids, this had no effect on the production of hNC spheroids (FIG. 4A).

### Example 4-2. Mobility analysis of hNC spheroids dependent upon concentrations of magnetic nanoparticles and confirmation of no relationship between concentration and mobility

The mobility of hNC spheroids produced by adding magnetic nanoparticles at a concentration of each of 100 µg/ml, 200 µg/ml, and 400 µg/ml using the method of Example 4-1 was analyzed. After culturing spheroids under each culture condition containing magnetic nanoparticles, the mobility of the spheroids was confirmed using a magnet.

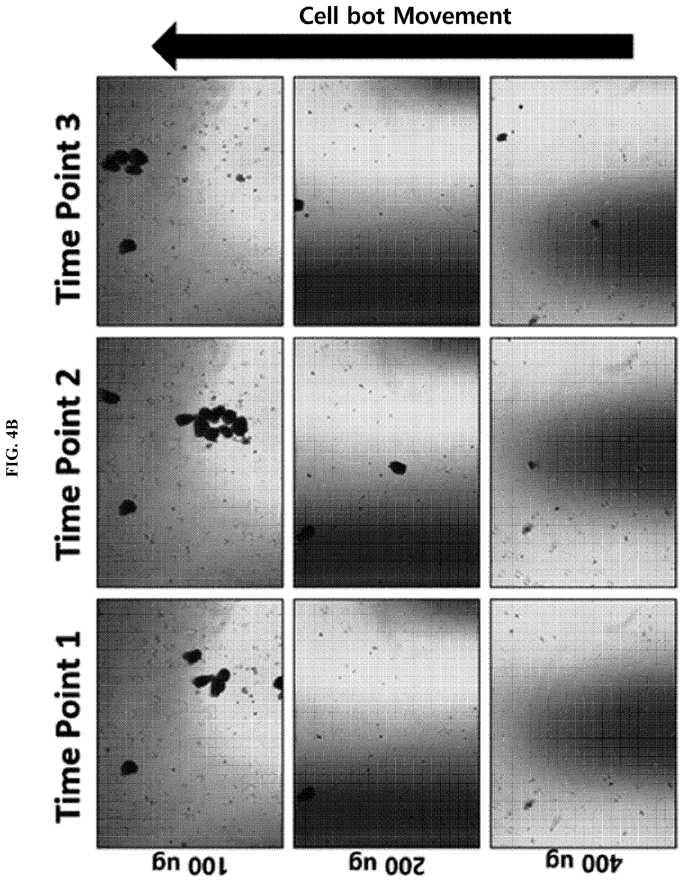

As a result, it was confirmed that the hNC spheroids produced from the hNCs internalizedwith the magnetic nanoparticles moved in the desired direction in a magnetic field regardless of the concentration of magnetic nanoparticles (FIG. 4B).

Therefore, the hNC spheroids produced with the hNCs internalized with the magnetic nanoparticles were proven to be able to move according to magnetism regardless of the incorporation amount of the magnetic nanoparticles, so the hNC spheroids internalized with the magnetic nanoparticles of the present invention were named "hNC spheroid cell bots.".

### Example 5. Establishment of method of producing hNC spheroid cell bots to reduce use concentration of magnetic nanoparticles

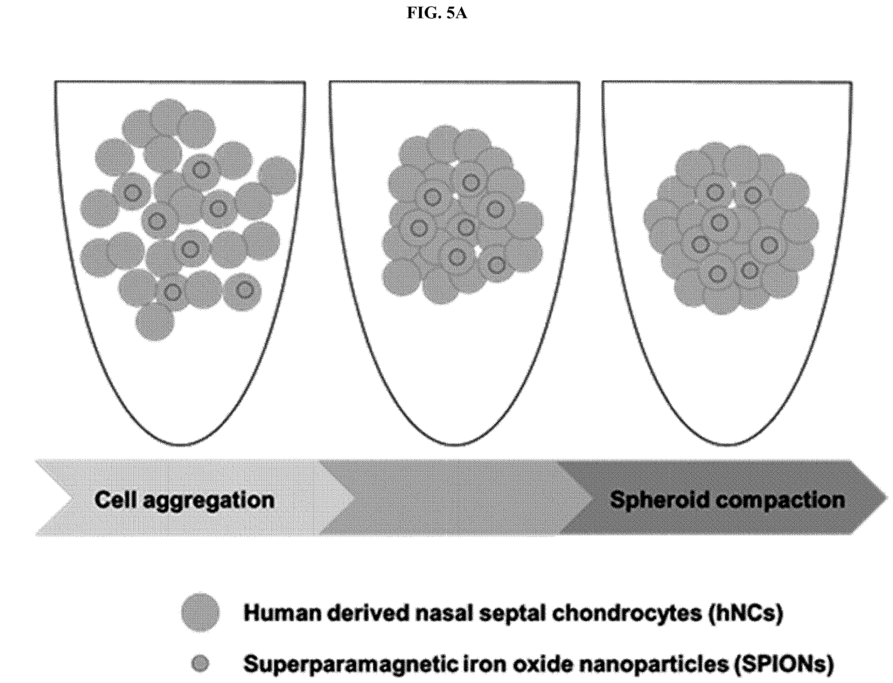

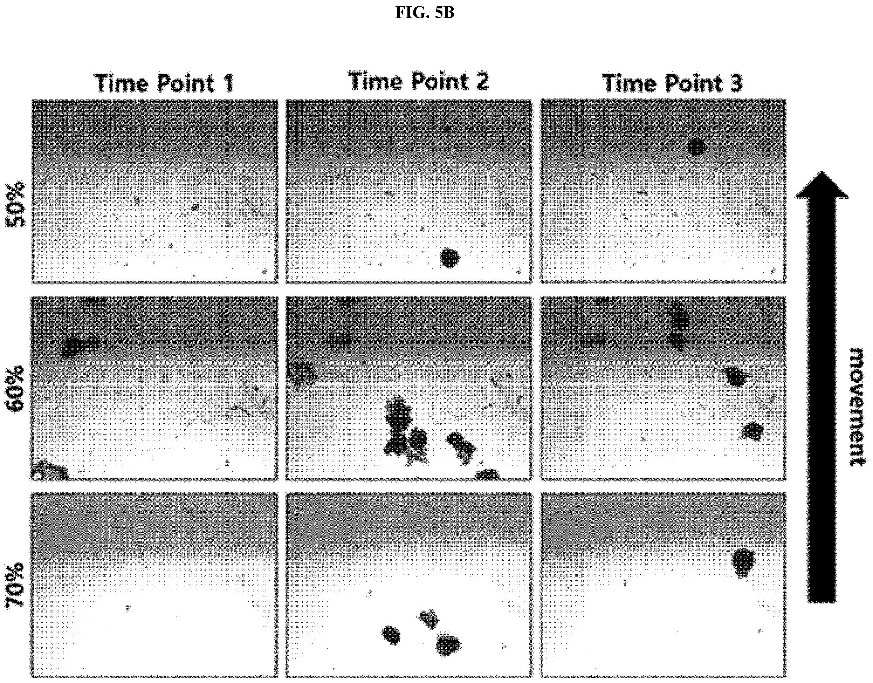

A new culture method that enables movement in a magnetic field and requires less magnetic nanoparticles than an existing production method by using both cell types internalized with and without magnetic nanoparticles in a spheroid production stage was developed (FIG. 5A).

Spheroids were cultured and produced by mixing hNCs without magnetic nanoparticles and hNCs internalized with 100 µg/ml of magnetic nanoparticles in a certain ratio. Here, the cell number ratios of hNCs and hNCs internalized with magnetic nanoparticles (hNCs:MNP_hNCs) were 50% (50:50), 60% (60:40), and 70% (70:30), respectively, and the hNC spheroid cell bots produced by mixing in the respective ratios were named 50%-mixed hNC spheroid cell bots, 60%-mixed hNC spheroid cell bots, and 70%-mixed hNC spheroid cell bots.

It was analyzed whether the mixed hNC spheroid cell bots produced in each ratio maintained their mobility in a magnetic field. Here, the experimental method was applied in the same way as in Example 4-2.

As a result, it was confirmed that mixed hNC spheroid cell bots moved smoothly under a magnetic field regardless of the mixing ratio.

### Example 6. Confirmation of excellent therapeutic activity of hNC spheroid cell bots for osteoarthritis

### Example 6-1. Establishment of animal model for osteoarthritis

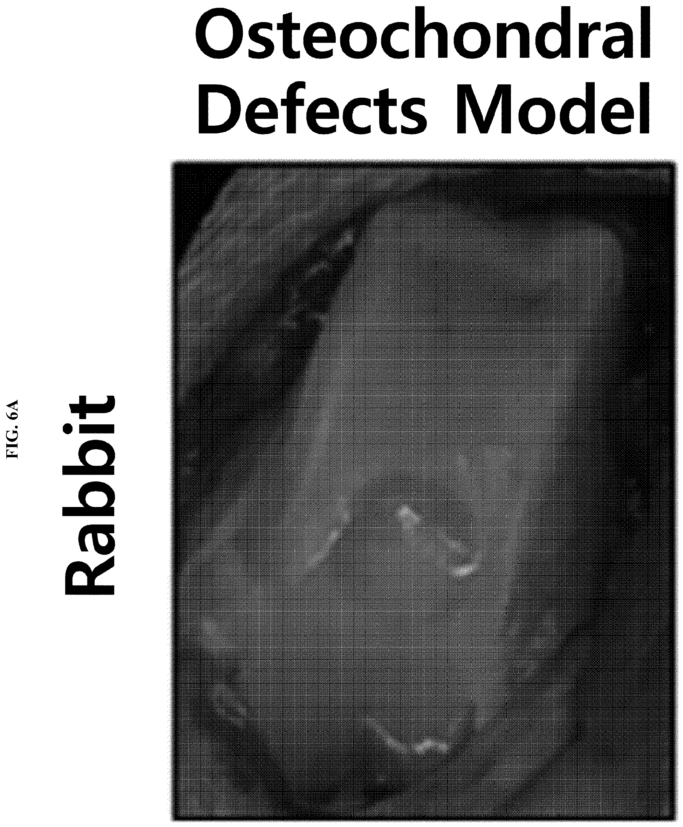

To evaluate the effectiveness of hNC spheroid cell bots for articular cartilage regeneration conducted by the Daegu-Gyeongbuk Advanced Medical Industry Promotion Foundation, a rabbit model of cartilage damage was first created. Specifically, rabbits were fasted for 1 day, and then anesthesia was induced by intramuscular administration of 5 mg/kg Alfaxalone. 2 to 4% isoflurane was used for inhalation anesthesia, and under anesthesia, a knee surgical site was shaved and applied with povidone (10%) + alcohol (70%) 3 times. After covering the area excluding the surgical site with a sterile drape, the skin, subcutaneous tissue, muscle, and synovial membrane of the knee area were incised. The patella was pushed outward to expose the distal end of the femur, and a 4-mm diameter circular defect was created in the cartilage of the intercondylar groove area using a high-speed drill to create an osteoarthritis animal model, a rabbit model of cartilage damage (FIG. 6A).

Next, hNC spheroid cell bots were produced to confirm therapeutic activity. Here, hNCs cells: hNCs cells internalized with 100 ug/ml magnetic nanoparticles were used in a ratio of 7:3 (70%:30%) to produce 70%-mixed hNC spheroid cell bots. Next, 50 to 80 ul of the 70%-mixed hNC spheroid cell bots were transplanted into the defect site of each animal of the osteoarthritis animal model at a concentration of 1x10^7 cells/ml to create an experimental group. After applying a tissue adhesive on the surgical site to fix it, the blood film, muscle, and skin were sutured, and the model was admitted after disinfecting the surgical site. A sham group and a negative control group were used as control groups. For each group, the following analysis was performed to confirm osteoarthritis treatment activity.

### Example 6-2. Analysis of clinical symptoms after transplantation with hNC spheroid cell bots

Clinical symptoms were observed for each group produced using the method of Example 6-1. To observe clinical symptoms, feed intake and body weight changes were checked for a total of 4 weeks.

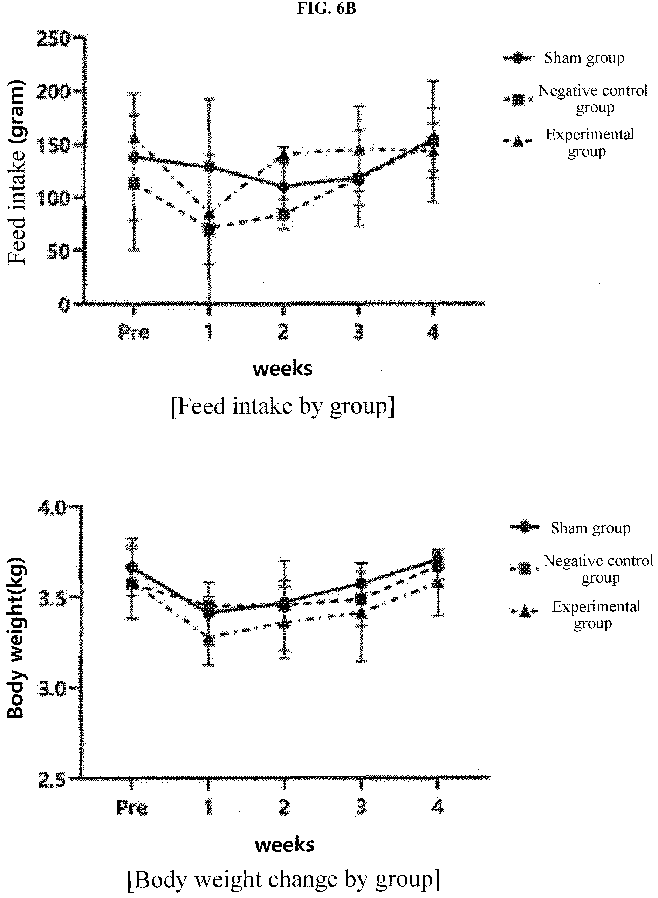

As a result (FIG. 6B), feed intake decreased in the negative control group and experimental group after surgery, but there was a tendency to recover in the second week, and there was no significant change in the sham group. In addition, there was no statistically significant difference between the groups in the weight measurement results, and no unusual findings were found in the clinical symptom observations and clinical pathology tests.

### Example 6-3. CT and histopathological analysis evaluation after transplantation with hNC spheroid cell bots

Histopathological analysis was performed on each group produced using the method of Example 6-1. Here, the rabbits corresponding to each group were euthanized, the femurs were removed, and micro-CT imaging was performed.

As a result, it was revealed that a hypoechoic material rather than bone partially restored the defect areas of the experimental group and the negative control group.

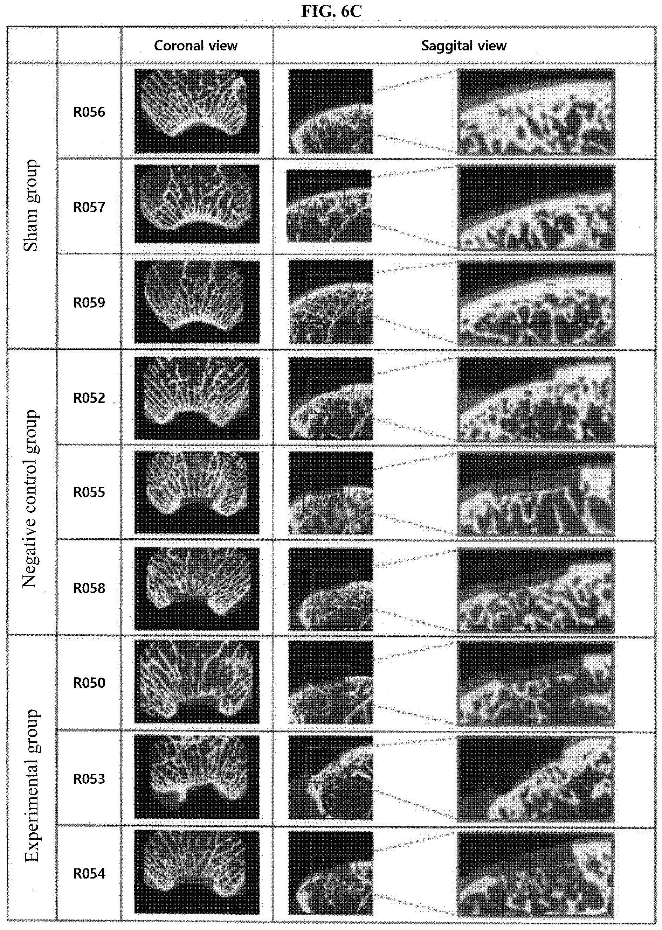

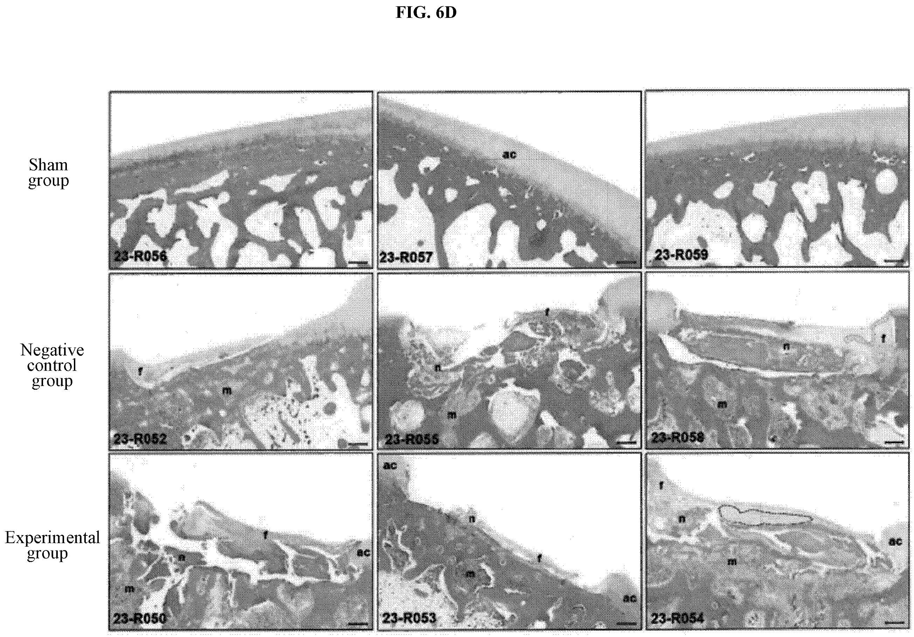

In addition, the histopathological examination revealed significant differences in the experimental group compared to the sham group, but no significant differences in the experimental group compared to the negative control group. When only the defect areas were checked, it was confirmed that the experimental group had a higher tendency to form cartilage tissue, compared to the negative control group (FIG. 6C and FIG. 6D).

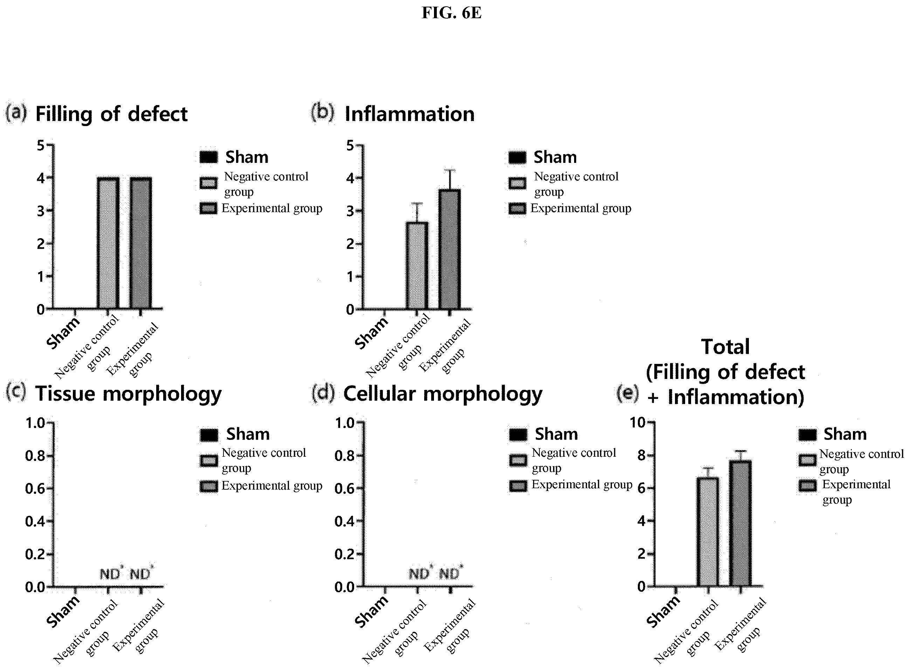

Quantitative analysis was performed by scoring the histopathological analysis results. As a result of the combined filing of defects and inflammation, a significant difference was confirmed in the experimental group compared to the sham group, and no significant difference was found in the experimental group compared to the negative control group (FIG. 6E).

The aforementioned description of the present invention is provided by way of example and those skilled in the art will understand that the present invention can be easily changed or modified into other specified forms without change or modification of the technical spirit or essential characteristics of the present invention. Therefore, it should be understood that the aforementioned examples are only provided by way of example and not provided to limit the present invention.

### [Industrial Applicability]

According to the pharmaceutical composition for treating cartilage damage disease comprising nasal septum chondrocytes internalized with magnetic nanoparticles as an active ingredient, a method of incorporating magnetic nanoparticles into nasal septum chondrocytes and a method of culturing the nasal septum chondrocytes internalized with magnetic nanoparticles to produce spheroids were established. It was confirmed that the nasal septum chondrocytes internalized with magnetic nanoparticles and the spheroids prepared therefrom by the method of the present invention exhibited therapeutic activity for cartilage damage while maintaining mobility in a magnetic field. In addition, even when spheroids are produced by mixing nasal septum chondrocytes internalized with magnetic nanoparticles and nasal septum chondrocytes, the above-described activity is maintained, and thus the amount of magnetic nanoparticles added may be significantly reduced, making the composition useful as a therapeutic composition for cartilage damage disease with excellent therapeutic effects without causing side effects, and thus has industrial applicability.

## Claims

1. A pharmaceutical composition for preventing or treating cartilage damage disease, comprising magnetic nanoparticle-internalized nasal septum chondrocytes (NSCs) as an active ingredient.

2. The pharmaceutical composition according to claim 1, wherein the magnetic nanoparticle-internalized nasal septum chondrocytes are spheroids.

3. The pharmaceutical composition according to claim 1, wherein, in the magnetic nanoparticle-internalized nasal septum chondrocytes, the magnetic nanoparticles are added in an amount of more than 0 and 800 µg per 1x10^6 nasal septum chondrocytes.

4. The pharmaceutical composition according to claim 2, wherein the spheroids are a mixture of nasal septum chondrocytes and the magnetic nanoparticle-internalized nasal septum chondrocytes,
wherein a number ratio of the nasal septum chondrocytes : the magnetic nanoparticle-internalized nasal septum chondrocytes is (5 to 7) : (3 to 5).

5. The pharmaceutical composition according to claim 1, wherein the composition is a pharmaceutical composition for injection, and
the injection is one selected from the group consisting of arthroscopic injection, and intra-articular injection.

6. The pharmaceutical composition according to claim 1, wherein the cartilage damage disease is one or more selected from the group consisting of osteoarthritis, degenerative arthritis, articular cartilage defect, rheumatoid arthritis, fracture, meniscus injury, muscle injury, ligament injury, plantar fasciitis, lateral epicondylitis, calcific myositis, traumatic joint or cartilage injury, and nonunion of fractures.

7. A kit for preventing or treating cartilage damage disease, comprising nasal septum chondrocytes (NSCs) internalized with magnetic nanoparticles.

8. A method of producing nasal septum chondrocytes internalized with magnetic nanoparticles, the method comprising:
(S1) producing nasal septum chondrocytes (NSCs) by treating nasal septum cartilage tissue, isolated from nasal septum tissue, with type 2 collagenase; and
(S2) culturing the nasal septum chondrocytes and additionally adding magnetic nanoparticles thereto.

9. A method of producing magnetic nanoparticle-internalized nasal septum chondrocyte spheroids for treating cartilage damage disease, the method comprising:
(S1) producing magnetic nanoparticle-internalized nasal septum chondrocytes by culturing nasal septum chondrocytes (NSCs), isolated from nasal septum tissue, and magnetic nanoparticles; and
(S2) adding the nasal septum chondrocytes and the magnetic nanoparticle-internalized nasal septum chondrocytes to a medium for culturing them in a spheroid shape.

10. A method of preventing or treating cartilage damage disease, the method comprising: administering a composition comprising the magnetic nanoparticle-internalizednasal septum chondrocytes (NSCs) as an active ingredient in a pharmaceutically effective amount to a subject in need thereof.

11. Use of a composition comprising magnetic nanoparticle-internalized nasal septum chondrocytes (NSCs) as an active ingredient for preventing or treating cartilage damage disease.

12. Use of a composition comprising the magnetic nanoparticle-internalized nasal septum chondrocytes (NSCs) as an active ingredient for producing a preparation for preventing or treating cartilage damage disease.
